# EUROPÄISCHE PATENTSCHRIFT

(11) **EP 2 747 792 B1**
(45) Veröffentlichungstag und Bekanntmachung des Hinweises auf die Patenterteilung: **25.04.2018**
(21) Anmeldenummer: 12750389.4
(22) Anmeldetag: 23.08.2012
(51) Int. Cl.: A61L 15/46, A61L 15/60

(54) **WUNDAUFLAGE AUFWEISEND EINEN ANTEIL AN KUPFER BZW. KUPFERIONEN**
WOUND DRESSING HAVING A CONTENT OF COPPER OR COPPER IONS
PANSEMENT COMPORTANT UNE CERTAINE PROPORTION DE CUIVRE OU D'IONS CUIVRE

(30) Priorität: 23.08.2011 DE 102011052941; 14.12.2011 DE 102011056429
(43) Veröffentlichungstag der Anmeldung: 02.07.2014
(73) Patentinhaber: BSN medical GmbH, 20253 Hamburg (DE)
(72) Erfinder: RIESINGER, Birgit, 48149 Münster (DE)
(74) Vertreter: Jostarndt Patentanwalts-AG
(86) Internationale Anmeldenummer: PCT/EP2012/066466
(87) Internationale Veröffentlichungsnummer: WO 2013/026912

(56) Entgegenhaltungen:
- EP-A1- 1 656 914
- EP-A2- 0 389 023
- WO-A1-2007/067111

## Beschreibung

Die vorliegende Erfindung betrifft eine Wundauflage aufweisend einen Anteil an Kupfer bzw. Kupferionen. Eine solche Wundauflage eignet sich insbesondere zur Aufnahme von Exsudat aus chronischen Wunden, wie z.B. bei Diabetes, Ulcus cruris und ähnlichen Erkrankungen auftreten.

Ebenso eignet sich eine solche Wundauflage auch für die Aufnahme von anderen Flüssigkeiten, insbesondere von Körperflüssigkeiten und -ausscheidungen.
Der Begriff "Exsudat" bezeichnet eine über die pathologischen Prozesse einer Wunde abgeleitete Wundflüssigkeit. So wie das Blut für den Transport von Nährstoffen und anderen Botenstoffen und damit für die Versorgung verschiedener Teile des Körpers verantwortlich ist, dient das Exsudat auf ganz ähnliche Weise der Versorgung des Wundbettes und der darin ablaufenden Heilungsprozesse. Um dieser Vielzahl an Funktionen gerecht zu werden, enthält es ein breites Spektrum an Komponenten, woraus ein spezifisches Gewicht resultiert, das leicht oberhalb dessen von Wasser liegt. Darin unterscheidet es sich auch vom Transsudat, welches ein deutlich geringeres spezifisches Gewicht mit einem geringen Zell- und Proteingehalt aufweist. Neben der Bereitstellung von Nährstoffen für die Fibroblasten und Epithelzellen beeinflusst das Exsudat die verschiedenen Prozesse der Wundheilung zeitlich und räumlich durch seinen hohen Gehalt an Wachstumsfaktoren und Zytokinen. Diese werden vor allem durch Thrombozyten, Keratinozyten, Makrophagen und Fibroblasten gebildet. Sie beeinflussen die Motilität, Migration und Proliferation der verschiedenen an der Wundheilung beteiligten Zellen. So wird das Einwandern von Zellen in den Wundgrund ebenso gefördert wie die Versorgung des neugebildeten Granulationsgewebes durch die Angiogenese. Auch die Wundreinigung wird durch das Exsudat unterstützt. Es enthält verschiedene Serin-, Cystein- und Aspartatproteasen sowie Matrix-Metalloproteasen, die in ihrer Aktivität streng reguliert Gewebe abbauen.

Bestandteile des physiologischen Exsudats sind insbesondere Salze, Glucose, Zytokine und Wachstumsfaktoren, Plasmaproteine, Proteasen (insbesondere Matrix-Metalloproteasen), Granulozyten und Makrophagen.

Kommt es nicht innerhalb einiger Wochen zu einer deutlichen Progression des Wundheilungsverlaufs entsprechend der verschiedenen Phasen der Wundheilung, so spricht man von einer chronischen Wunde. Dabei betrachtet man jedoch andauernde exsudative Phasen als Komplikation und spricht von einer pathologischen Exsudation, welche zu einer Chronifizierung der Wunde beitragen kann. Die zugrunde liegenden Ursachen sind meist komplex und können durchaus auch systemischer Natur sein. Es überrascht jedoch aufgrund der zuvor erläuterten Bedeutung des Exsudats für die Wundheilung nicht, dass sich Komplikationen der Wundheilung in einer deutlich veränderten Zusammensetzung und Wirkung des Exsudats widerspiegeln.

Insbesondere bei chronischen, schlecht heilenden Wunden, wie sie z.B. bei Diabetikern häufig anzutreffen sind, spielen Infektionen eine große Rolle. Dieses Problem hat sich in der jüngeren Vergangenheit durch antibiotikaresistente pathogene Keime verschärft. Insbesondere multiresistente Keime wie z.B. Vancomycin-resistente Enterokokken, Methicillin-Resistente Staphylococcus aureus und multiresistente Pseudomonas aeruginosa spielen in der klinischen Praxis heute eine oftmals fatale Rolle.

So werden in den USA jährlich bei 90.000 Ulcus-Cruris-Patienten mit Diabetes Gliedmaßen amputiert, da diese aufgrund infektiöser Komplikationen durch multiresistente Keime nicht anders behandelbar sind.

Bei Inkontinenzartikeln, insbesondere bei Windeln, besteht hingegen die Gefahr, das sich insbesondere Vancomycin-resistente Enterokokken, die in einem solchen Artikel, wenn dieser benutzt wird, ideale Lebensbedingungen vorfinden, vermehren und z.B. wunde Hautstellen der den Artikel tragenden Person befallen. Dies ist insbesondere deswegen eine reelle Gefahr, da Inkontinenzpatienten häufig auch bettlägerig sind und z.B. einen Dekubitus aufweisen (beispielsweise im Steißbereich), bei welchem sich bei Einwanderung solcher resistenter Keime eine äußerst schwer zu behandelnde Infektion ausbilden könnte. Überdies ist bei solchen Patienten aufgrund ihrer mitunter langen Krankengeschichte die Gefahr der Existenz Vancomycin-resistenter Enterokokken im Verdauungstrakt gegeben.

Aber auch bei anderen Hygiene- und/oder Wundpflegeartikeln wie z.B. Monatsbinden, Tampons, Inkontinenzartikeln, Windeln, Kranken- oder Liegeunterlagen, Stomabeuteln, Stomabeuteleinlagen, Fußmatten, OP-Tüchern, Redonflaschen, Taschentüchern und/oder Hyperhidrose-Artikeln besteht die Gefahr, dass sich antibiotikaresistente pathogene Keime aufgrund der oftmals günstigen Wachstumsbedingungen in denselben vermehren und eine Infektionsgefahr darstellen.

Kupfer wird seit Jahrhunderten zur Desinfizierung von Flüssigkeiten, Feststoffen und Geweben verwendet. Kupfer ist ein Übergangsmetall und gehört als schwach reaktives Schwermetall zu den Edelmetallen.

Gegenwärtig kommt Kupfer beispielsweise bei der Wasseraufbereitung, als Algenvernichtungsmittel, Fungizid, Nematozid, Molluskizid sowie als ein antibakterielles und Bewuchsschutzmittel zum Einsatz, denn im Gegensatz zur geringen Empfindlichkeit des menschlichen Gewebes gegenüber Kupfer reagieren Mikroorganismen äußerst empfindlich auf Kupfer.
Im Gegensatz zu Antibiotika sind bislang keine Mikroorganismen bekannt, die einer konstanten Exposition gegenüber Kupfer beständig sind. Dies liegt wahrscheinlich daran, dass Kupfer simultan verschiedene zellspezifische Ziele der Mikroorganismen angreift.
Die WO 2006/048879 beschreibt ein Verfahren zur Behandlung von Blasen und Läsionen in dem ein Material enthaltend wasserunlösliche Kupferverbindungen, die Kupfer(I)-Ionen, Kupfer -(II)-ionen oder Kombinationen derselben bei Kontakt mit einer Flüssigkeit freisetzt. Ebenso beschreibt die WO 01/74166 ein antimikrobielles und antivirales Polymermaterial aufweisend Kupferionen. Es wird gezeigt, dass Fasern aus dem Polymermaterial aufweisend Kupferionen in vitro antimikrobielle und antivirale Eigenschaften entfalten. Allerdings ist sowohl das Verfahren als auch das Polymermaterial zur Behandlung von tiefen, exudierenden chronischen und/oder langsam heilenden Wunden ungeeignet.
Dies ist insbesondere deswegen der Fall, da sowohl das beschriebene das Verfahren als auch das Polymermaterial einen häufigen Verbandswechsel bei derartigen Wunden nach sich ziehen. Dieser Wechsel des Verbandmaterials stört jedoch die Wundheilung und ist insbesondere bei chronischen Wurden traumatisch und für den Patienten oft äußerst schmerzhaft.
Zudem passt sich das beschriebe Polymermaterial bzw. das Material enthaltend wasserunlösliche Kupferverbindungen nicht der Körperkontur des Patienten an. Somit sind Wundpflegeartikel aus diesen Materialen vor allem dann, wenn sie über einen längeren Zeitraum auf der Wunde verbleiben sollen, um den eben erwähnten Nachteil der Wundheilung zu vermeiden, von Nachteil. In EP1656914 wird ein Einweg-Absorptionskörper zum Aufsaugen von aus den Wunden austretendem, flüssigen Exsudat beschrieben, aufweisend: - eine flüssigkeitsdurchlässige Hülle und wenigstens einen flüssigskeitabsorbierenden Textilabschnitt mit darin vorhandenen Superabsorber-Teilchen, der in der Hülle untergebracht ist und wobei der Absorptionskörper eine Menge an eine antimikrobiell wirkenden Substanz wie z.B. eine kupferhaltige Substanz aufweist.

Aufgabe der vorliegenden Erfindung ist es, eine Wundauflage bereit zu stellen, der einige der oben genannten Nachteile nicht aufweist. Diese Aufgabe wird mit einer Wundauflage gemäß dem vorliegenden Hauptanspruch gelöst; die Unteranspruche geben bevorzugte Ausführungsformen an.

Der Begriff "Wundauflage" soll im Folgenden bevorzugt eine flächige Wundauflage oder ein Wundpflegetuch bezeichnen, die für den humanmedizinischen, dentalmedizinischen und/oder veterinärmedizinischen Sektor gedacht ist.
Besagter Wundpflegeartikel kann bevorzugt in Form eines Tupfers, eines Wundtuchs, einer Wundauflage, einer Wundkompresse, eines Wundpolsters, einer ggf. elastischen Wickel, einer Bandage, eines Pflasters oder eines Strumpfes vorliegen.

Insbesondere kann der Begriff "Wundauflage" auch als ein Ensemble verschiedener Produkte verstanden werden, die in einer gegebenen Anordnung auf der zu behandelnden Wunde angeordnet werden. Dieses Ensemble kann eine physikalische Einheit bilden, indem die verschiedenen Produkte in einer gemeinsamen Hülle zusammengefasst oder - ggf. ohne Hülle - adhäsiv miteinander verbunden sind. Das Ensemble kann jedoch auch in Form eines Kits vorliegen, bei dem die verschiedenen Produkte ggf. mithilfe einer Wickel, eines Klebebandes, eines Klebestreifens oder eines Pflasters in der gegebenen Anordnung auf der zu behandelnden Wunde angeordnet sind.

Der Begriff "Kranken- oder Liegeunterlage" bezeichnet solche Produkte, die im Pflege- oder Krankenhausbereich als Unterlagen für Patienten oder Pflegebedürftige Personen verwendet werden und austretende Flüssigkeiten aufnehmen sollen.

Kupfer findet sich in Enzymen des Energiestoffwechsels und ist wichtig für die Bildung des roten Blutfarbstoffes (Hämoglobin) sowie für die Funktion der Zytochrome in den Mitochondrien. Kupfer ist auch wichtig für die Aufnahme von Eisen aus der Nahrung. Beim Kupfermangel treten daher Symptome des Eisenmangels und Funktionsminderungen der entsprechenden Enzyme auf.

Nicht zuletzt aufgrund des überwältigenden Erfolgs der Ende der 1930ger Jahre eingeführten Antibiotika ließ das Interesse an der Verwendung von Kupfer für Hygienemaßnahmen nach.

Mit dem Aufkommen der oben erwähnten multiresistenten Keime hat auch das Interesse an der Verwendung von Kupfer für Hygienemaßnahmen wieder zugenommen.

So wurde in einem erfolgversprechenden Versuch der Asklepios Klinik Wandsbek in Hamburg eine komplette Krankenhausstation mit Türgriffen, Türplatten und Lichtschaltern aus Kupfer ausgestattet. Hintergrund war, dass Mikroorganismen nicht nur von Hand zu Hand, sondern in vielen Fällen auch über das Berühren von Klinken und Schaltern übertragen werden.

Kupfer deaktiviert bzw. tötet höchst effizient ein breites Spektrum an Viren und Mikroorganismen. Dabei wirkt Kupfer bzw. die Kupferionen auf verschiedene Arten.
1) Da sie hoch redoxaktive Metallionen darstellen, fördern sie die Peroxidation von Membranlipiden und beschädigen dadurch die Zellwand des Mikroorganismus. In dieser Eigenschaft bewirkten sie zudem eine Verschiebung der essentiellen Metalle von ihren ursprünglichen Bindungszentren und stören so den Stoffwechsel des Mikroorganismus.
2) Sie beschädigen das genetische Material des Mikroorganismus, indem sie die DNA binden und somit ihre Konformation ändern
3) Die beschädigen die Proteine des Mikroorganismus, insbesondere bewirken sie einen Abbau von Sulfhydrylgruppen sowie eine Oxidation bestimmter Aminosäurereste.
4) Sie beeinträchtigen die oxidativen Phosphorylierung und stören das osmotische Gleichgewicht.
Zudem beeinflusst Kupfer bzw. Kupferionen die unterschiedlichen biologischen Verteidigungsmechanismen des Mikroorganismus derart, dass eine Resistenz gegen antimikrobielle Wirkstoffe praktisch nicht mehr vorhanden ist.
Somit weist die erfindungsgemäße Wundauflage den Vorteil auf, dass er durch Kupfer bzw. Kupferionen sowohl antimikrobielle Eigenschaften als auch antivirale Eigenschaften besitzt und gleichzeitig - durch die hydroaktiven Polymere - sich der Körperkontur des Verwenders in amorpher Weise anpasst. Dies erhöht insbesondere bei langsam heilenden, exudierenden, chronischen und/oder tieferen Wunden den Tragekomfort erheblich.
Neben Kupfer ist auch Silber für seine antimikrobillen Eigenschaften bekannt. Allerdings weist Silber im Gegensatz zu Kupfer den Nachteil auf, dass es durch Silberresorption über die Haut zu einer Silberakkumulation im Organismus kommt.
Diese kann neben der Argyrie, einer irreversiblen schiefergrauen Verfärbung von Haut und Schleimhäuten, zu Geschmacksstörungen, Geruchsunempfindlichkeit sowie zerebralen Krampfanfällen führen. Auch in Gefäßen und inneren Organen wie Leber, Nieren und Milz setzt sich Silber ab. Im Zusammenhang damit sind chronische Oberbauch-Schmerzen und Schwindel beschrieben worden. Daher stufte die FDA 1999 in den USA freiverkäufliche Arzneimittel sowohl für die innerliche als auch äußerliche Anwendung, die Silbersalze oder kolloidales Silber enthalten, als bedenklich ein, und es wurde eine eigene behördliche Verwaltungspraxis geschaffen.

Somit weist die erfindungsgemäße Wundauflage den Vorteil auf, dass er durch Kupfer bzw. Kupferionen sowohl antimikrobielle Eigenschaften als auch antivirale Eigenschaften besitzt und gleichzeitig - durch die hydroaktiven Polymere - sich der Körperkontur des Verwenders anpasst. Dies erhöhte insbesondere bei langsam heilenden, exudierenden, chronischen und/oder tieferen Wunden den Tragekomfort erheblich.

Ein weiterer Vorteil einer erfindungsgemäßen Wundauflage liegt darin, dass dieser durch die Quellfähigkeit der hydroaktiven Polymere länger auf der Wunde verbleiben kann als herkömmliche Wundauflagen.
Grundsätzlich ist gerade dann, wenn der Kolonisierungsgrad einer Wunde nicht genau bekannt ist, ein häufiger Verbandwechsel erforderlich, um im Fall einer Infektion schnell eingreifen zu können. Die erfindungsgemäße Wundauflage macht einen solchen häufigen Wechsel verzichtbar, da er die Kontrolle von Infektionen ermöglicht, und trägt so dem Bedürfnis nach Verlängerung der Wundruhephasen Rechnung, wie sie von Fachleuten im Rahmen der modernen Wundpflege insbesondere bei Diabetes-spezifischen chronischen Wunden gefordert wird.
Da Kupfer ein lebensnotwendiges Spurenelement ist, das die Hautschichten stabilisiert, ist es für die Haut bzw. bei der Verwendung auf Wunden unbedenklich. Darüber hinaus zeigen jüngere Forschungen, dass Kupfer bzw. Kupferionen sogar die Wundheilung fördern indem sie das Wachstum von Kapillaren und die Regenration der Epidermis anregt und die Bildung von Kollagen, Fibronektin, Elastin, Integrin und anderer Schlüsselproteinen initieren bzw. stimulieren.
Unter dem Begriff "antimikrobielle Eigenschaften" wird im Folgenden eine Substanz, die die Vermehrungsfähigkeit oder Infektiosität von Mikroorganismen reduziert oder sie abtötet beziehungsweise inaktiviert also beispielsweise bakterizid wirkt, verstanden.

Unter dem Begriff "Mikroorganismen" werden im Folgenden mikroskopisch kleine Organismen verstanden. Beispiele für Mikroorganismen sind Bakterien, Pilze, mikroskopische Algen und Protozoen.

Unter dem Begriff "antivirale Eigenschaften" werden im Folgenden Substanzen verstanden, die gegen Viren und die durch sie hervorgerufenen Erkrankungen gerichtet sind. Im Allgemeinen hemmen sie die Vermehrung von Viruspartikeln im Körper, wirken also virostatisch. Antivirale Substanzen greifen häufig in enzymatische Prozesse ein, die zur Vermehrung der Viruspartikel notwendig und gleichzeitig spezifisch für das betreffende Virus sind. Ein weiterer Wirkungsmechanismus antiviraler Substanzen besteht darin, die Anlagerung und das Eindringen der Viruspartikel in die Zelle zu verhindern. Außerdem kann die Reifung neuer Viruspartikel oder deren Ausschleusung verhindert werden, so dass keine weiteren Zellen befallen werden können und sich die Infektion nicht weiter ausbreiten kann.

Vorzugsweise handelt es sich bei den Kupferionen um Kupferkationen. In einer Ausführungsform handelt es sich bei den Kupferionen um Kupfer(I)- und/oder Kupfer(II)-ionen, also einwertigte oder zweiwertige Kupferionen. In einer weiteren Ausführungsform handelt es sich bei den Kupferionen um gebundene Kupferionen. Unter dem Begriff "gebundene Kupferionen" wird im Folgenden verstanden, dass die Kupferionen zunächst als Verbindung mit einem anderen Stoff vorliegen, beispielsweise als Salz (im folgenden als Kupfersalze bezeichnet), und somit nach außen keine Ladung tragen. Beispiele solcher gebundener Kupferionen stellen Kupfersulfat, Kupferoxychlorid, Kupferchlorid und/oder Kupferhydroxid dar.

In einer Ausführungsform lösen sich die gebundenen Kupferionen durch Kontakt mit Wundflüssigkeit bzw. Exsudat aus der Verbindung und entfalten sodann ihre antimikrobiellen und antiviralen Eigenschaften. Selbstverständlich ist es jedoch auch möglich, dass Kupferionen, während sie als Verbindung mit einem anderen Stoff vorliegen, ihre antimikrobiellen und antiviralen Eigenschaften entfalten.
Die gebundenen Kupferionen bzw. elementares Kupfer haben den Vorteil, dass je nach Anwendung die Kupferionen einfacher in die erfindungsgemässe Wundauflage integriert werden können, da sie weniger reaktiv sind. Besonders bevorzugt handelt es sich bei den gebundenen Kupferionen um Kupferoxid. Unter dem Begriff "Kupferoxid" wird im Folgenden Kupfer(I)-oxid, Kupfer(II)-oxid oder eine Mischung der beiden Kupferoxide verstanden.
Vorzugsweise liegt das Kupfersalz, bevorzugt das Kupferoxid, in Teilchen mit einer Größe zwischen 0,5µm und 20µm vor. Besonders bevorzugt in einer Größe zwischen 1µm und 5µm vor.

In einer Ausführungsform liegt das Kupfer bzw. die Kupferionen in Pulverform, in Granulatform, in Drahtform, als Kolloid und/oder als Späne vor. Es werden Fasern vorgesehen, die mit elementarem Kupfer imprägniert sind oder in welche Drähte, Litzen oder Filamente bestehend aus elementarem Kupfer eingearbeitet sind. Unter dem Begriff "imprägniert" wird im Folgenden verstanden, dass Fasern z.B. aus Acryl während ihrer Herstellung d.h. vor der Extrusion mit ≥ 0,1% bis ≤ 15% bis Kupferionen gemischt werden. Beispielsweise können die Kupferionen in Form eines Kolloids vorliegen. Unter dem Begriff "Kolloid" werden im Folgen Kupferionenteilchen verstanden, die in einem anderen Medium (Feststoff, Gas oder Flüssigkeit), dem Dispersionsmedium, fein verteilt sind. Das einzelne Kolloid ist typischerweise zwischen 1 nm und 1 µm groß. Sind sie beweglich (etwa in einem flüssigen Dispersionsmedium), so zeigen Kolloide meist Brownsche Bewegung.
Besagtes Kolloid kann beispielsweise in Form einer Salbe auf den Hygiene- oder Pflegeartikel aufweisend einen Anteil an hydroaktiven Polymeren aufgebracht sein.

Unter dem Begriff "hydroaktive Polymere" sollen superabsorbierende Polymere (SAP) verstanden werden, die feuchtigkeitsbindende Eigenschaften haben.

Die genannten hydroaktiven Polymere nehmen wässrige Wundexsudate auf und bilden dabei ebenfalls eine feuchte Oberfläche aus. Ggf. nehmen sie eine gelartige Form an. Die feuchte Oberfläche bzw. die Gelform trägt dazu bei, die Wundhaftneigung des Wundpflegeartikels zu reduzieren, so dass dieser sich nach Verwendung atraumatisch und schmerzlos ablösen lässt. Durch die Gelform weist der Wundpflegartikel eine kühlende und somit scherzlindernde Wirkung auf. Überdies ermöglicht die Gelform die Ausbildung eines wundheilungsfördernden Feuchtklimas.

Superabsorbierende Polymere (SAP) sind Kunststoffe, die in der Lage sind, ein Vielfaches ihres Eigengewichts - bis zum 1000-fachen - an Flüssigkeiten aufzusaugen. Chemisch handelt es sich dabei um ein Copolymer aus Acrylsäure (Propensäure, C₃H₄O₂) und Natriumacrylat (Natriumsalz der Acrylsäure, NaC₃H₃O₂), wobei das Verhältnis der beiden Monomere zueinander variieren kann. Zusätzlich wird ein so genannter Kernvernetzer (Core-Cross-Linker, CXL) der Monomerlösung zugesetzt, der die gebildeten langkettigen Polymermoleküle stellenweise untereinander durch chemische Brücken verbindet (sie "vernetzt"). Durch diese Brücken wird das Polymer wasserunlöslich. Beim Eindringen von Wasser oder wässrigen Salzlösungen in die Polymerpartikeln quellen diese auf und straffen auf molekularer Ebene dieses Netzwerk, so dass das Wasser ohne Hilfe nicht mehr entweichen kann.

Alternativ können die Superabsorber auf Methylacrylsäurebasis, Acrylamidopropansulfonsäure-Copolymere, Stärke-Acrylnitril-Pfropfpolymere, Stärke-Acrylsäure-Pfropfpolymere, Vinylacetat-Acrylsäureester-Copolymere, Acrylnitril- oder Acrylamid-Copolymere gewählt sein. Ebenso kann es sich bei besagten hydroaktiven Polymeren auch um Hydrogel-Nanopartikel aufweisend Hydroxy-Terminierte Methacrylatmonomere, wie 2-Hydroxyethylmethacrylat (HEMA) und/oder 2-Hydroxypropylmethacrylat (HPMA), die z.B. als Altrazeal vermarktet werden, handeln.

Die superabsorbierenden Polymere können in der erfindungsgemäßen Wundauflage in Form von Fasern, eines Fasergewirkes, -geleges oder -vlieses und/ oder einer Faserwatte vorliegen. Die Superabsorber-Teilchen können in Pulver- oder Granulatform mit einer Partikelgröße zwischen 100 µm und etwa 1000 µm vorliegen. Es handelt sich bevorzugt um eine Wundauflage enthaltend superabsorbierende Polymere, die in ihrem Aufbau etwa den im Handel erhältlichen Produkten "sorbion sachet", "Tenderwet", "Vliwasorb" und/oder "Curea P" entspricht.

Bevorzugt kann dabei vorgesehen sein, dass die Wundauflage als Spülkörper fungiert. Hiezu ist vorgesehen, dass die Wundauflage vor Aufbringen auf die Wunde mit einer physiologischen Lösung (z.B. 0.9 % Kochsalz, Ringerlösung oder dergleichen) getränkt wird oder entsprechend getränkt vorkonfektioniert vorliegt. Eine solche Wundauflage gibt während der Verwendung kontinuierlich Flüssigkeit an die Wunde ab, spült diese und nimmt Exsudat, Zelltrümmer, nekrotische Bestandteile, Bakterien, Debris und dergleichen auf.
Wie die Anmelderin in früheren Patentanmeldungen gezeigt hat, sind superabsorbierende Polymere in der Lage, große Mengen an Exsudat aufzunehmen und zu binden. Sie reduzieren so den Anteil an pathologischem Exsudat in der Wunde und fördern damit die Wundheilung.

Die besagten superabsorbierenden Polyacrylate binden jedoch nicht nur Flüssigkeiten, sondern auch Bakterien, Proteine und andere Biomoleküle.
Dieser Effekt ergänzt sich ideal mit dem antimikrobiellen und antiviralen Effekt des Kupfers bzw. Kupferionen. Dies ist der Fall, da Studien gezeigt haben, dass Kupfer bzw. Kupferionen ihre antimikrobiellen und antiviralen Eigenschaften innerhalb kürzester Zeit entfalten. Dadurch aber kann es - vor allem wenn es sich bei den Mikroorganismen um Bakterien handelt - zu einer Freisetzung von Endotoxinen und bakterielle Pathogenitätsfaktoren (insbesondere bakterielles Hämolysin und Leukocidin) kommen. Diese wiederum können Entzündungen, Allergien, Schocks (insbesondere anaphylaktische Schocks und/oder das Toxic Shock Syndome) und Fieber erzeugen (Herxheimer-Reaktion). Dies gilt insbesondere dann, wenn Kupferoxid topisch in Form einer Wundauflage aufgebracht wird. Eine erfindungsgemäße Wundauflage, aufweisend einen Anteil an superabsorbierenden Polymeren, und einen Anteil an Kupfer bzw. Kupferionen, vorzugsweise Kupferoxid, trägt also nicht nur dazu bei, die Anzahl an pathogenen Erregern in der Wunde durch Aufnahme der Mikroorganismen insbesondere der Bakterien mittels SAP und/oder durch Lysis der Bakterien zu reduzieren, sondern nimmt auch aktiv die entstandenen Lysate auf, und zwar insbesondere die darin enthaltenen Endotoxine.
Letzteres trägt auch zu einer Entlastung des Immunsystems bei, das ansonsten die entstehenden Endotoxine handhaben und entsorgen müsste.
Auch in Bezug auf den Abbau von hartnäckigen Biofilmen lässt sich ein Synergismus beschreiben. So können die Superabsorber Biofilme austrockenen, so dass Risse entstehen, durch welche Kupfer- oder Kupferionen ihre bakterizide Wirkung ausüben können. Kupfer ist bekannt für seine biofilmabbauende Wirkung, die insbesondere im Rohrleitungsbau von Bedeutung ist. Dies ergänzt sich überdies mit den bakteriostatischen und bakteriziden Eigenschaften von Superabsorbierenden Polymeren sowie deren Potential zur Modulation von Gewebeschädlichen Proteasen (Bindung, Inaktivierung und Entfernung).

Ein weiterer Vorteil ergibt sich für solche erfindungsgemässe Wundauflage, die nicht im unmittelbaren Wundkontakt stehen, wie beispielsweise Kranken- oder Liegeunterlagen. Die Löslichkeit von Kupfer und Kupfersalzen wie Kupfer-I-oxid und Kupfer-II-oxid ist stark pH-Wert abhängig. Im leicht sauren Bereich, wie er durch die Anwesenheit von auf Polyacrylaten basierenden Superabsorbern generiert wird, setzt das Produkt vermehrt Kupfer bzw. Kupferionen frei, welche dann für die Inaktivierung der in den aufgenommenen Körperflüssigkeiten enthaltenen Bakterien und Mikroorganismen zur Verfügung steht (gewollter Burst). Ferner kann gewährleistet sein, dass nach Flüssigkeitsaufnahme durch Ansteigen des pH-Werts die Löslichkeit des Kupfers bzw. der von Kupfer und Kupfersalzen wieder zurückgeht und/oder die verstärkt auftretenden Acrylat-Anionen die freigesetzten Kupferionen an die Zellulose-SAP-Matrix binden.

Ein weiterer Vorteil einer erfindungsgemäßen Wundauflage liegt darin, dass sie nicht nur zur Behandlung von Wunden einsetzbar ist, die mit antibiotiokaresistenten Keimen infiziert sind, sondern dass er auch in solchen Fällen einsetzbar ist, in welchen eine Antibiotikatherapie aus anderen Gründen nicht angezeigt ist. Dies trifft z.B. zu
- für Schwangere, die in der Regel nur mit einem sehr eingeschränkten Antibiotikaspektrum (insbesondere Erythromycin) behandelt werden dürfen
- für Personen mit einer Antibiotikaallergie
- für Personen mit Leberschäden oder systemischen Defekten, bei denen bei einer Antibiotikatherapie eine metabolische Intoxikation zu befürchten ist

Ein weiterer Vorteil der erfindungsgemäßen Wundauflage ist, dass sie sich insbesondere für die Behandlung von Verbrennungswunden eignet, die einerseits stark exsudieren (was den Einsatz von SAP sinnvoll macht), andererseits nicht oder kaum noch durchblutet werden, was eine systemische Antibiotikatherapie erschwert. Verbrennungswunden sind jedoch äußert anfällig für Infektionen, insbesondere für die im Krankenhausbereich häufig anzutreffenden multiresistenten Keime, so dass hier eine Therapie mit Kupfer bzw. Kupferionen eine neue, erfolgversprechende Option darstellt.

Ein weiterer Vorteil der erfindungsgemäßen Wundauflage liegt in einer synergistischen Reduktion der entzündungsbedingten Geruchsbildung, wie sie bei chronischen Wunden häufig anzutreffen ist. Durch die Aktivität des Kupfers bzw. der Kupferionen wird die für die Geruchsbildung verantwortliche Stoffwechselaktivität der Bakterien, die insbesondere Buttersäure freisetzen, gemindert. Gleichzeitig nehmen die superabsorbierenden Polymere sowohl bereits entstandene Geruchsstoffe als auch Exsudat und Wasser aus der Wunde auf und binden diese. Durch die Trockenlegung der Wunde werden überdies die Wachstumsbedingungen für die geruchserregenden Bakterien verschlechtert, was zu einer weiteren Geruchsminderung führt. Die erfindungsgemäße Wundauflage ist besonders vorteilhaft weil elementares Kupfer bzw. die Kupfer(I)- und Kupfer(II)-Ionen gemischt mit hydroaktiven Polymeren in Form von superabsorbierenden Polymeren vorliegen. Denn durch die von den SAP aufgenommene Wundflüssigkeit bzw. das Exsudat kann sich ein Kolloid ausbilden. In diesem können die beispielsweise als Faserimprägnierung vom Wunde- und/oder Pflegeartikel bereitgestellten Kupferionen, insbesondere wenn es sich um eine Mischung aus Kupfer(I)- und Kupfer(II)-Ionen handelt, durch eine Redoxreaktion zwischen dem stabileren Kupfer(II)-Ion und dem stärker antiviral wirkendem Kupfer(I)-Ion wechseln. Dies hat den Vorteil, dass der erfindungsgemäßen Hygiene- oder Pflegeartikel durch die anwesenden Kupfer(II)-Ionen länger lagerfähig ist.
Die vorteilhafte Ausbildung eines Kolloids kann selbst dann auftreten, wenn die Kupferionen zuvor als gebundene Ionen z.B. als Kupfer(I)- und/oder Kupfer(II)-oxid oder sogar in elementarer Form vorlagen. Dies scheint an der Zusammensetzung des Wundexsudats zu liegen, in dem sich die Kupferionen leichter aus dem Kupferoxid lösen, als in reinem Wasser.
Diese vorteilhafte Ausbildung eines Kolloids überrascht, da die SAPs bekannt dafür sind, Exsudat so effektiv zu binden, dass eigentlich keine Flüssigkeit zur Ausbildung des Kolloids vorhanden sein sollte. Offenbar reicht jedoch die Gelbildung der superabsorbierenden Polymere aus um die Kollidbildung und somit den vorteilhaften Übergang des Kupfers bzw. der Kupferionen zu ermöglichen. Diese implizite und überraschende Art der Kolloidbildung hat gegenüber bewusst hinzugefügten Kolloiden z.B. in Form von Salben, die auf den erfindungsgemäßen Hygiene- oder Pflegeartikel aufgetragen werden, den Vorteil, dass die Herstellung sehr viel effizienter und kostengünstiger erfolgen kann. Da elementares Kupfer bzw. Kupferionen beispielsweise gebunden als Kupferoxid einfach mit den superabsorbierenden Polymeren während der Herstellung des erfindungsgemäßen Hygiene- oder Pflegeartikel gemischt werden können oder auf andere Weise eine räumliche Nähe der beiden Anteile sichergestellt werden kann. Beispielsweise könnten Fasern aus superabsorbierenden Polymeren einfach mit elementarem Kupfer bzw. Kupferionen z.B. in Form von Kupferoxid imprägniert werden.

Alternativ ist vorgesehen, dass es sich bei den Superabsorbierenden Polymere in Faser-, Garn-, Watte- Vlies-, Gewirke-, Airlaid-, Nonwoven- und/oder Gewebeform handelt.
Der Begriff "Airlaid" bezeichnet einen speziellen Vliesstoff aus Zellstoff und Polyolefinfasern, in den ggf. superabsorbierende Polymere eingebettet sind.
Besonders bevorzugt liegen die superabsorbierenden Polymere in Form eines Absorptionskörpers aufweisend ein Vlies enthaltend Zellulosefasern vor.

Der Absorptionskörper kann bevorzugt einen im Wesentlichen flachen Absorptionskörper aus Absorptionsmaterial aufweisen, der aus einem aufsaugenden Vlies mit darin verteilten superabsorbierenden Polymeren in Form von Fasern besteht. Dieser Absorptionskörper kann der absorbierenden Einlage entsprechen, die in einer Wundauflage der Anmelderin der vorliegenden Erfindung enthalten ist, wie sie beispielsweise in der WO03094813, der WO2007051599 und der WO0152780 offenbart ist. Der Absorptionskörper kann in einer anderen Ausgestaltung ebenso einen Kern bilden, der - ggf. flockenartige - Fasern oder Garne aus superabsorbierenden Polymeren sowie superabsorbierenden Polymeren in Granulatform aufweist, wobei die Granulate an die Fasern bzw. Garne in mehreren Höhen angeklebt bzw. angeschweißt sind, und die Granulate über mehr als 50 % der gesamten Bauhöhe wenigstens eines Abschnitts des Kerns verteilt sind, wobei vermengte Bereiche von Granulat und Fasern vorliegen. Der Gewichtsanteil der superabsorbierenden Polymeren kann dabei bevorzugt im Bereich zwischen 10 und 100 Gew.-% liegen. Besonders bevorzugt sind dabei Werte von 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 und/oder 100 Gew.-%

Besonders bevorzugt weist der Absorptionskörper ein Vlies, bevorzugt ein Nonwoven oder Airlaid, auf, das aus Superabsorbierenden Polyacrylat- Fasern ("SAF") besteht oder diese als Bestandteil enthält. Die Fasern können beispielsweise mit Fluff Pulp (Zellulose) oder mit Polyesterfasern gemischt sein. Alternativ oder Zusätzlich kann ein Schichtaufbau vorgesehen sein.
Der Absorptionskörper kann in einer anderen Ausgestaltung ebenso mindestens eine flache Lagen aufweisend Fasern oder Garne aus superabsorbierenden Polymeren enthalten, an welche superabsorbierende Polymere in Granulatform geklebt sind. Dadurch ergibt sich in einer bevorzugten Ausgestaltung ein Aufbau des Körpers, der wenigstens zweiSchichten aufweist, wobei mindestens eine Deckschicht eine Schicht aufweisend superabsorbierende Polymere unterlegt. Optional kann eine zweite, flankierende Deckschicht vorgesehen sein.
Dabei liegen in der Ebene keine Vermengungen von Fasern und superabsorbierenden Polymeren vor; sondern lediglich fixierte Benachbarungen beider Materialien. Die ggf. vorgesehenen mehreren Lagen können dabei in einer bevorzugten Ausgestaltung auch durch Walzen, Pressen, Kalandrieren oder ähnliche Verfahren physisch miteinander verdichtet sein. Weiterhin kann auch ein Material vorgesehen sein, in welchem die Superabsorbierenden Polymere nicht in eine Zellulose-Matrix eingebracht sind, sondern ein amorphes Material aufweisend Superabsorbierende Polymere, das beispielsweise zwischen zwei Lagen eingebettet ist, die aus einem Vlies aufweisend Zelluose oder ein anderes Material bestehen Die in dem vorliegenden vorgeschlagene Verwendung von Superabsorbierenden Polymeren in Faser- oder Garnform weist gegenüber partikulären superabsorbierenden Polymeren eine Reihe von Vorteilen auf:
i) So haben besagte Fasern oder Garne einen Dochteffekt. Auf diese Weise können sie bei Kontakt mit einer Flüssigkeit sehr viel schneller diese Flüssigkeit aufnehmen und binden als dies partikuläre superabsorbierende Polymere können.
ii) überdies können die Flüssigkeitssströme lenken bzw. richten. Auf diese Weise kann z.B. eine Wundrandmazeration verhindert werden.
iii) Sie können anders als partikuläre superabsorbierende Polymere zu Vliesen, Geweben oder ähnlichem verarbeitet werden. Auf diese Weise kann z.B. die Hülle einer Wundauflage aus diesen Fasern oder Garnen gefertigt sein, und die superabsorbierenden Eigenschaften können so sehr viel näher an den Wundgrund gebracht werden.
iv) Da in vielen Fällen auf ein gesondertes Trägermaterial verzichtet werden kann, kann der Anteil an superabsorbierenden Materialien in einem Hygiene- oder Pflegeartikel, insbesondere einem Wundpflegeartikel erheblich erhöht werden; im Extremfall kann er sogar einen Gewichtsanteil von 100 % annehmen.
v) Besagte Fasern oder Garne bzw. die daraus hergestellten Produkte besitzen eine sehr viel höhere Weichheit und eine geringere Abrasivität als die entsprechenden partikulären superabsorbierenden Polymere.
vi) Besagte Fasern oder Garne lassen sich zu einem Gefüge verarbeiten, ohne dass - wie es bei partikulären superabsorbierenden Polymeren erforderlich ist - ein Kleber oder ein Schweissverfahren verwendet werden muß. Dies hat sowohl in Bezug auf die Reinheit des Produkts als auch in Bezug auf die Pharmakologie und etwaige Allergenität erhebliche Vorteile.
vii) Im Gegensatz zu partikulären superabsorbierenden Polymere läßt sich die Dimensionierung besagter Fasern oder Garne sehr viel genauer steuern und kontrollieren, was einerseits zu Verhinderung von Stäuben führt, wie sie bei Verwendung von partikulären superabsorbierenden Polymere häufig entstehen., und was andererseits die Produktqualität (Homogenität und Reproduzierbarkeit) wesentlich erhöht.
viii) Aufgrund der fehlenden Ausbildung von Stäuben kann ggf. auch auf die Verwendung einer gesonderten Hülle verzichtet werden.
ix) Besagte Garne oder Fasern können in aus dem Stand der Technik bekannten Airlaids, die partikuläre superabsorbierenden Polymere enthalten, die Trägerfasern des Airlaids ersetzen, um so den Anteil an superabsorbierenden Materialien in einem Hygiene- oder Pflegeartikel, insbesondere einem Wundpflegeartikel und damit die gesamte Absorptionskapazität - zu erhöhen.
Jegliche zwei- oder dreidimensionale Anordnung der Fasern oder Garne ist hier denkbar. So können die Fasern oder Garne gerichtet oder ungerichtet (Wirr-Warr), in mehreren Lagen oder sonstwie angeordnet sein.
Superabsorbierende Fasern aus Polyacrylaten werden z.B. von der Firma Technical Absorbents unter dem Handelsnamen "Oasis Super Absorbent Fibre" angeboten und vertrieben. Sie weisen wie alle Superabsorbierenden Polyacrylate eine sehr hohe Aufnahmekapazität für Flüssigkeiten auf. Diese Fasern können z.B. in Form eines Vlieses, eines Airlaids, eines Gewebes, eines Airlaids und/oder eines Nonwoven vorliegen. Solche Fasern sind z.B. aus der DE 69807337 bekannt.
Bevorzugt ist weiterhin vorgesehen, dass die in Faserform vorliegenden superabsorbierenden Polymere mindestens teilweise in Form einer Watte, eines Vlieses, eines Airlaids und/oder eines Nonwoven vorliegen.
Hierzu werden bevorzugt Fasern mit mittleren Längen von 5 - 50 mm verwendet. Die Herstellung erfolgt mit bekannten Methoden, wie z.B. dem Kardieren oder dem Airlaid-Verfahren. Dabei können die Superabsorbierenden Fasern alleiniger Bestandteil des jeweiligen Materials sein. Bevorzugt ist jedoch vorgesehen, dass die Wundauflage überdies einen Anteil an stützenden Fasern aufweist, die auch in feuchtem Zustand die Integrität der Wundauflage gewährleisten, wobei besagte Fasern ausgewählt sind aus der Gruppe enthaltend
a) Cellulosefasern,
b) Viskosefasern,
c) Hohlfasern
d) Alginatfasern, und/oder
e) Polyester-, Polyolefine-, Polyurethan-, Polyvinylalkohol- oder Polymaidfasern, bzw. Mischungen oder Copolymere derselben
Hierzu können insbesondere auch sogenannte Bonding-Points beitragen, wie sie durch örtlich spezifiziertes Herbeiführen von sogenannten "Hydrogen-Bonding"-Bindungen erzeugt werden.
Auf diese Weise lassen sich die Absorptionseigenschaften des betreffenden Materials, aber auch andere Eigenschaften wie z.B. Dehnbarkeit, Reißfestigkeit, Verhalten bei Durchnässung und dergleichen, genau steuern.
Dabei kann insbesondere vorgesehen sein, dass ein Gewebe vorgesehen ist, bei dem z.B. die Kettfäden aus superabsorbierenden Garnen gefertigt sind, während die Schussfäden aus anderen Garnen bestehen, die z.B. stützende Fasern gemäß der obigen Aufzählung enthalten. Ein solches Gewebe weist gerichtete hydroaktive Eigenschaften auf, d.h. es nimmt Flüssigkeit in einer Richtung auf und leitet sie weiter.

In einer Ausführungsform weist die erfindungsgemäße Wundauflage mindestens einen Bestandteil aus der Gruppe umfassend Aktivkohle und Silberionen auf.
Dies hat den Vorteil, dass die antimikrobiellen und geruchsbindenden Eigenschaften des Kupfers bzw. der Kupferionen weiter unterstützt werden.

In einer Ausführungsform weist die erfindungsgemäße Wundauflage einen Materialabschnitt aus superabsorbierenden Polymeren und eine mindestens abschnittsweise um diesen Materialabschnitt angeordnete Hülle auf.
Vorzugsweise besteht eine ggf. vorgesehene Hülle zumindest teilweise auch aus einem hydrophobem Material, beispielsweise aus Polypropylen oder aus einem hydrophob ausgerüsteten Naturmaterial, wie Baumwolle. Die hydrophoben Eigenschaften der Hülle verhindern das Verkleben mit der Wundoberfläche und tragen dazu bei, dass das Wundexsudat schneller ins Innere der Hülle gelangen kann.
Die Hülle kann auch aus einem anderen Kunststoff, insbesondere einer Polyurethan- oder Polyethylenfolie oder aus künstlicher Spinnenseiden Folie hergestellt sein.

Bevorzugt ist überdies vorgesehen, dass die Hülle einer erfindungsgemäßen Wundauflage zumindest abschnittsweise eine adhäsive Beschichtung aufweist, und zwar bevorzugt auf der wundabgewandten Seite, mit deren Hilfe sie - z.B. mit einem Verband - im Wundbereich fixiert werden kann.
Weiterhin kann auch vorgesehen sein, dass die Hülle einen über die eigentliche Wunde überstehenden Bereich aufweist, der mit Klebestreifen zur Fixierung versehen ist.
Vorzugsweise sind der Kupfer bzw. die Kupferionen und/oder die superabsorbierenden Polymere innerhalb der Hülle angeordnet, sie können aber auch in bzw. auf der Hülle angeordnet oder Bestandteil derselben sein. Dies ist insbesondere dann möglich, wenn ein flächiges Hüllenmaterial in Form einer Bahn verwendet wird, das mindestens abschnittsweise Kupfer oder Kupferionen enthält. Hierzu können Fasern, die mit Kupfer oder Kupferionen versehen oder imprägniert werden (wie andernorts hierin beschrieben) verwendet werden, um daraus - ggf. mit anderen Fasern, wie gewebten oder vliesartig zusammengesetzten Kunstfasern, wie Polypropylen- oder Polyethylenfasern, aber auch Baumwolle, Seide oder Viskose gebildet - Garne, Vliese, Gewebe oder Gewirke herzustellen, aus denen dann das besagte flächige Hüllenmaterial hergestellt werden kann. Dieses, so ist bevorzugt vorgesehen, kann dann verwendet werden, um einen Absorptionskörper aufweisend superabsorbierendes Material zu umhüllen. Kupfer oder Kupferionen können jedoch auch in eines der in dieser Anmeldung beschriebenen Vliesmaterialien enthaltend superabsorbierende Polymere eingebracht sein.
Kupfer bzw. die Kupferionen kann jedoch auch in oder auf ein Matrixmaterial ein- oder aufgebracht sein. Dies kann beispielsweise durch Oberflächenbeschichtung mittels PECVD (Plasma Enhanced Chemical Vapor Deposition), CVD (chemical vapor deposition) oder Dip Coating erzeugt werden, oder aber durch Einbringen in das Material mittels Coextrusion oder zeitgleichem Verspinnen oder Filzen von kupferhaltigen und nicht kupferhaltigen Fasern oder Garnen.
Bevorzugt weist das Matrixmaterial einen Kunststoff- oder einem Kunststofffaseranteil auf. Hierbei kann es sich beispielsweise um Polyester, PLA Polyester, Nylon, Polypropylen, Polyethylen oder Latex handeln.

Der Kupferanteil kann dabei im Bereich zwischen 0,1 und 20 Gew.-%, liegen, bevorzugt zwischen 1 und 10 Gew.-%, besonders bevorzugt zwischen 2 und 5 Gew.-%. Ganz besonders bevorzugte Werte sind insbesondere 1, 2, 3, 4, 5, 6, 7, 8, 9, 10, 11, 12, 13, 14, 15, 16, 17, 18, 19 oder 20 Gew.-%

Vorzugsweise weist die Hülle der Wundauflage ein hydrophobes Material auf, bzw. das Hüllenmaterial ist hydrophob ausgerüstet. Die hydrophoben Eigenschaften der Hülle verhindern das Verkleben mit der Wundoberfläche und tragen dazu bei, dass die Wundexsudat-Partikeln schneller ins Innere der Hülle gelangen können.

Dabei kann vorgesehen sein, dass die Hülle zumindest abschnittsweise ein elastisches Material aufweist, so z.B. Fasern aus Lycra oder Elasthan. Auch so ist gewährleistet, dass der Materialabschnitt bei Flüssigkeitsaufnahme in seinem Volumen zunehmen kann und nicht durch die Hülle eingeschränkt ist.

Besagte Hülle umgibt den Absorptionskörper, bildet eine Barriere gegen feste Ausscheidungen bildet und ermöglicht den Durchtritt von anderen ausgetretenen Substanzen zu dem innerhalb der Hülle angeordneten Materialabschnitt aus Absorptionsmaterial. Die Hülle ist bevorzugt wenigstens teilweise von einer Naht abgeschlossen

Die Poren oder Maschen der Hülle sind bevorzugt 0,05 mm bis 1,0 mm, vorzugsweise 0,20 mm bis 0,50 mm groß. Grundsätzlich kann dabei vorgesehen sein, dass die mittlere Porengröße geringer ausfällt als die mittlere Größe der Partikel enthaltend hydroaktive Polymere.

Weiterhin kann bevorzugt vorgesehen sein, dass die Poren oder Maschen durch Materialabschnitte begrenzt sind, welche im Schnitt durch die Hülle etwa bogenförmig sind und mit ihren Bogen-Scheiteln nach außen zeigen. Dies gilt sowohl für Nonwovenmaterialien als auch für Folienmaterialien

Absorptionskörper der genannten Art - jedoch ohne Kupfer - sind beispielsweise in der WO03094813 und der WO2007051599 der Anmelderin der vorliegenden Erfindung offenbart.

Das Material der Hülle kann derart strukturiert sein, dass die Hülle eine raue Innenfläche und eine glatte Außenfläche aufweist. Vorzugsweise ist die raue Innenfläche der Hülle durch trichterförmige Perforationen gebildet, die sich jeweils in Richtung Innenfläche verjüngen und in eine freie Öffnungskante ("Auskragung") auslaufen. Diese raue Innenfläche wirkt den Verschiebungen des Inhaltes der Hülle entgegen, so dass auf eine Fixierung mit Klebepunkten verzichtet werden kann. Dementsprechend kann die glatte Außenfläche des Hüllenmaterials durch gewölbte, sich zwischen den Perforationen erstreckende Materialabschnitte gebildet sein. Ein solches Hüllenmaterial kann im Gegensatz zu einem beidseitig ebenen als "dreidimensional" bezeichnet werden.
Besonders bevorzugt ist dabei vorgesehen, dass das besagte dreidimensionale Hüllenmaterial auf das zuvor erwähnte Polypropylenvlies auflaminiert ist. Eine solche Ausgestaltung hat verbesserte Flüssigkeitsaufhahmeeigenschaften zur Folge.
Ebenso kann vorgesehen, sein, dass der Wundpflegeartikel auf seiner wundabgewandten Seite einen flüssigkeitsundurchlässigen Wäscheschutz ("Backsheet") aufweist.
Besonders bevorzugt weist der Absorptionskörper in Draufsicht auf seine Flachseite eine Fläche (F1) auf, welche in seinem nicht benetzten Zustand um 3% bis 75% kleiner als die Fläche (F2) der flachgelegten Hülle ist und in der Hülle frei beweglich oder fixiert ist, wobei die Hülle ganzflächig Poren aufweist, welche jeweils kleiner als die unbenetzten superabsorbierenden Polymere sind.
Bevorzugt ist vorgesehen, dass dieses Flächenverhältnis 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, und/oder 75 % beträgt.
Auf diese Weise ist gewährleistet, dass der Materialabschnitt bei Flüssigkeitsaufnahme in seinem Volumen zunehmen kann und nicht durch die Hülle eingeschränkt ist.
Besonders bevorzugt ist vorgesehen, dass die Hülle in Draufsicht auf seine Flachseite einen umlaufenden, über die Naht hinausragenden Überstand aufweist und der Absorptionskörper frei von harten, scharfen Kanten und Ecken ist. Die Wundauflage weist Kupfer bzw. Kupferionen in Form von Kupferoxid mit einer Mindestteilchengröße von ≥1 µm auf, das zusammen mit den superabsorbierenden Polymeren im Materialabschnitt angeordnet ist. Der Materialabschnitt weist zumindest an seiner der Wunde zugewandten Seite eine Hülle auf. Die Poren dieser Hülle weisen Größen von ≤ 1µm auf.
Da die meisten Bakterien einen Durchmesser zwischen etwa 0,1 und 700 µm haben, hat dieser Wundpflegeartikel den Vorteil, dass die Bakterien durch die Hülle gelangen können, wo das Kupferoxid seine antimikrobielle sprich bakterizide Wirkung entfalten und die SAPs die Bakterien bzw. die von ihnen freigesetzten Toxine binden. Das Kupferoxid seinerseits kann aber nicht in die Wunde gelangen, so es nicht vom Körper resorbiert werden kann. Zwar ist die Resorption von Kupfer über eine Wunde, wie oben erwähnt, generell unbedenklich, allerdings weist die soeben beschriebene Wundauflage, bei der es nicht zur Resorption kommen kann, trotzdem eine bei Patienten höhere Akzeptanz auf.
In einer bevorzugten Ausführungsform umschließt die Hülle den Materialabschnitt aus superabsorbierenden Polymeren der erfindungsgemäßen Wundauflage. Weiterhin kann die Hülle auf der, der Wunde abgewandten Seite flüssigkeitsundurchlässig ausgestaltet sein.

In einer weiteren Ausführungsform weist die erfindungsgemäße Wundauflage
a) einen Abschnitt aufweisend Weichschaumstoff, und/oder
b) ein Wunddistanzgitter auf, und/oder
c) eine Wunddrainageeinrichtung zum Einsatz bei Unterdruck.
Bei dem Abschnitt aus Weichschaumstoff kann es sich um mindestens ein Material ausgewählt aus der Gruppe enthaltend thermoplastische Weichschäume, wie Polyurethan-, Polyamid- oder Polyetherschaum, Silikonschaum sowie Cellulose-Schaum oder Naturschwamm handeln.

Der Begriff "Wunddistanzgitter", bezeichnet ein gitter- oder gazeartiges Gebilde, dass - häufig als sogenannter "Primärverband" - unmittelbar auf eine Wunde aufgelegt wird, bevor ggf. ein Sekundärverband aufgelegt wird. Wunddistanzgitter werden häufig auch als "Wundgazen" bezeichnet und dienen primär dazu, ein verkleben der gesamten Wundauflage mit der Wunde zu verhindern. Hierfür sind Wunddistanzgitter aus einem Material gefertigt oder mit einem solchen beschichtet, dass eine Haftung an der Wunde reduziert bzw. verhindert. Alternativ kann auch die Geometrie des Wunddistanzgitters so ausgestaltet sein, dass eine Haftung an der Wunde reduziert bzw. verhindert wird.

Bevorzugt ist ferner vorgesehen, dass der Primärverband aus einem Silikon oder aus einem mit Silikon beschichteten Material bestehen oder dieses aufweisen kann. So kann z.B. ein Gitter, das aus einem Silikon oder aus einem mit Silikon beschichteten Material besteht oder dieses aufweist, vorgesehen sein, oder eine perforierte oder mit Poren versehene Folie, ein perforiertes oder Poren ausbildendes Vlies oder Gewebe oder ein Gitter aus einem Kunststoffmaterial - beispielsweise Polyethylen, Polypropylen oder Polyamid - vorgesehen sein, dass mit einem Silikon mindestens einseitig beschichtet ist.

Das Silikon kann so ausgebildet sein, dass es dezidiert adhäsiv wirkt, dergestalt, dass der Primärverband an der Wunde haftet, gleichfalls aber eine Haftung des ggf. häufig zu wechselnden Sekundärverbandes an der Wunde, oder gar eine Eingranulation desselben, verhindert. Die Haftungseigenschaften von Silikon können technisch sehr genau eingestellt werden, sodass ein sicheres Haften gewährleistet werden kann, ohne dass ein Ablösen des Primärverbandes Schmerzen - etwa durch anhaftende Körperbehaarung - erzeugt oder gar traumatisch wirkt.

Ferner kann auch ein Kupfer- oder Kupferionenhaltiger Schaumstoff, bevorzugt offenzellig vorgesehen sein, der mit superabsorbierenden P=polymeren versehen ist. Ein solcher Schaum kann beispielsweise in einer Unterdruck-Vorrichtung Verwendung finden.

Weiterhin kann der erfindungsgemäße Hygiene- oder Pflegeartikel auch in ein Wundversorgungssystem zur Wunddrainage unter Einsatz von Unterdruck eingebracht sein. Solche Systeme sind z.B. in den Schriften DE202004017052, WO2006048246 und DE202004018245 der Anmelderin der vorliegenden Erfindung offenbart. Das Flächengewicht des erfindungsgemäßen Hygiene- oder Pflegeartikels kann dabei im Bereich zwischen 50 und 2000 g/m² liegen. Bevorzugt sind dabei Flächengewichte von 50, 100, 150, 200, 250, 300, 350, 400, 450, 500, 550, 600, 650, 700, 750, 800, 850, 900, 950, 1000, 1050, 1100, 1150, 1200, 1250, 1300, 1350, 1400, 1450, 1500, 1550, 1600, 1650, 1700, 1750, 1800, 1850, 1900, 1950, und/oder 2000 jeweils +/- 25 g/m² vorgesehen.

Die Dicke kann dabei im Bereich zwischen 1 und 50 mm liegen. Bevorzugt sind dabei Dicken von 2, 4, 6, 8, 10, 12, 14, 16, 18, 20, 22, 24, 26, 28, 30, 32, 34, 36, 38, 40, 42, 44, 46, 48, und/oder 50 jeweils +/- 1 mm vorgesehen.

Die Aufnahmekapazität kann dabei im Bereich zwischen 3 und 5000 ml 0.9 % Kochsalz/m² bei 0,2 psi Druck liegen. Bevorzugt sind dabei Werte von 100, 200, 300, 400, 500, 600, 700, 800, 900, 1000, 1100, 1200, 1300, 1400, 1500, 1600, 1700, 1800, 1900, 2000, 2100, 2200, 2300, 2400, 2500, 2600, 2700, 2800, 2900, 3000, 3100, 3200, 3300, 3400, 3500, 3600, 3700, 3800, 3900, 4000, 4100, 4200, 4300, 4400, 4500, 4600, 4700, 4800, 4900, 5000, 0.9 % Kochsalz/m² vorgesehen.

Alternativ kann die Aufnahmekapazität dabei im Bereich zwischen 2 und 100 g Wasser/g liegen. Bevorzugt sind dabei Werte von 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 und/oder 100 g Wasser/g.

Der Gesamtgehalt an Superabsorbierenden Polymeren kann dabei im Bereich zwischen 5 und 100 % w/w liegen. Bevorzugt sind dabei Werte von 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 und/oder 100 % w/w.

Die Zugfestigkeit kann dabei im Bereich zwischen 5 und 80 N/5 cm liegen. Bevorzugt sind dabei Werte von 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79 und/oder 80 N/5 cm.

Die Dehnbarkeit kann dabei im Bereich zwischen 3 und 80 % liegen. Bevorzugt sind dabei Werte von 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79 und/oder 80 %.

Überdies kann die erfindungsgemäße Wundauflage, oder der darin befindliche Absorptionskörper, Körper sich wiederholende Musterungen oder Maserungen aufweisen, wie z.B. ein Karomuster, ein Stanzmuster oder dergleichen.

Besonders bevorzugt ist ferner vorgesehen, dass ein in besagter erfindungsgemäßer Wundauflage befindlicher Absorptionskörper neben einer Lage aufweisend hydroaktive Polymere mindestens eine zweite flankierende Lage aufweist, die weniger oder keine hydroaktiven Polymere aufweist und flächenmäßig über erstgenannte hinausragt. Auf diese Weise wird sichergestellt, dass die Lage aufweisend hydroaktive Polymere entsprechend der Flüssigkeitsaufnahme an Volumen gewinnen kann, ohne dass die Volumenzunahme nach außen hin erkennbar ist, weil letztere durch die zweite Schicht kaschiert wird.

Ferner weist die erfindungsgemäße Wundauflage bevorzugt eine Hülle auf, die wiederum Mittel aufweist, die so gestaltet und/oder ausgewählt sind, dass die Hülle zumindest teilweise durch eine durch Flüssigkeitsaufnahme bedingte Volumenzunahme des Absorptionskörpers zielgerichtet verformbar ist. Grundsätzlich kann ein solches Verhalten mit unidirektional elastischem Material erzielt werden, also einem Material, das in einer Richtung dehnbar ist, in der orthogonal hierzu liegenden Richtung jedoch nicht.

Ferner ist bevorzugt vorgesehen, dass die erfindungsgemäße Wundauflage eine Zusammensetzung, enthaltend mindestens einen nutritiven, mindestens einen desinfizierenden bzw. dekontaminierenden und/oder mindestens einen Proteasen hemmend wirkenden Wirkstoff und/oder Wirkstoffkomplex zur äußeren Versorgung und/oder Behandlung von Wunden des menschlichen oder tierischen Körpers aufweist.

Unter dem Begriff "chronische Wunden" sollen Wunden verstanden werden, die nicht primär auf traumatische Einwirkungen zurückgehen. Zwar können traumatische Einwirkungen der ursprüngliche Auslöser einer solchen Wunde gewesen sein, aber die chronische Wunde zeichnet sich vor allem durch eine verzögerte Wundheilung auf. Chronische Wunden weisen häufig - wenn überhaupt - nur leichte Blutungen auf, dafür oftmals eine starke Exsudation.
Unter dem Begriff "leichte Blutung" soll eine Blutung verstanden werden, die nicht arteriellen Ursprungs ist, sondern ggf. venösen Ursprungs oder interstitiellen bzw. kapillaren Ursprungs, und die in jedem Fall so leicht ausfällt, dass sie nicht mittel- oder unmittelbar lebensbedrohend ist.

Unter dem Begriff "akut blutende Wunden" sollen solche Wunden verstanden werden, die zu großen Blutverlusten führen. In der Regel sind hierfür arterielle Blutungen verantwortlich, die z.B. durch traumatische Einwirkungen verursacht werden. Akut blutende Wunden können u.U. mittel- oder unmittelbar lebensbedrohend sein. Aus diesem Grunde hat bei akut blutenden Wunden die Blutungsstillung eine sehr hohe Priorität.

Weiterhin ist die Verwendung einer Wundauflage gemäß einem der vorherigen Ansprüche als Kranken- und/oder Liegeunterlage vorgesehen. Hier kommen insbesondere die oben genannten Vorteile beim Release aktiven Kupfers bei leicht erniedrigtem pH-Wert zum Tragen. Der Begriff "Kranken- oder Liegeunterlage" bezeichnet solche Produkte, die im Pflege- oder Krankenhausbereich als Unterlagen für Patienten oder Pflegebedürftige Personen verwendet werden und austretende Flüssigkeiten aufnehmen sollen.
Dabei kann insbesondere vorgesehen sein, dass eine solche Kranken- und/oder Liegeunterlage eine im wesentlichen flächige Gestalt aufweist, wobei vorzugsweise an mindestens einem ihrer Enden in Form eines Materialumschlags eine Tasche ausgebildet ist, in welche beispielsweise die Füße eines Pateinten oder Pflegebedürftigen eingebracht werden können. Alternativ kann eine solche Tasche auch für die Entsorgung der benutzen Unterlage verwendet werden (indem die gesamte Unterlage nach Benutzung in die besagte Tasche eingewickelt wird), oder aber zur Aufnahme von Hygieneprodukten, wie beispielsweise Urinbeuteln, Windeln und dergleichen Verwendung finden.
Diese Tasche kann beispielweise durch randständig ausgebildete Kleb- oder Schweißnähte fixiert werden.

Bevorzugt kann weiterhin die Unterseite solche Kranken- und/oder Liegeunterlagen aus einem atmungsaktivem Material bestehen, welches reißfest und wasserdicht ist, gleichzeitig aber Luft und ggf auch Wasserdampf passieren lässt. Textile Materialine mit solchen Eigenschaften (beispielsweise durch eine Mikroporöse Struktur erzielt) sind auf dem Markt bekannt, beispielsweise aus dem Wundversorgungsbereich oder dem Bereich textiler Funktionsmaterialien in der Sportbekleidung.

Die besagte Kranken- und/oder Liegeunterlage kann durch Ihre Dotierung mit Kupfer oder Kupferionen aber auch
- die Ausbildung eines entzündlichen Dekubitus vorbeugen
- dazu beitragen, einen bakteriellen Biofilm abzubauen, und/oder
- die Ausbildung von Gerüchen hemmen.

In allen drei Fällen kommt ein Synergismus mit den genannten Superabsorbierenden Polymeren ins Spiel. Im ersten Fall nehmen letztere die Exsudate und/oder Körperausscheidungen auf und verhindern so die Mazeration an den Wundrändern, im zweiten Fall legen letztere den Biofilm trocken, so dass Risse entstehen, durch welche Kupfer- oder Kupferionen ihre Biofilmabbauende Wirkung ausüben können, und im letzteren Fall wird die aufgenommene Flüssigkeit desinfiziert, sodass keine Gerüche entstehen können.

Weiterhin ist die Verwendung eines erfindungsgemäßen Hygiene- oder Pflegeartikels als Kompressionsbandage vorgesehen. Solche Bandagen kommen beispielsweise in der Venenkompressionstherapie zum Einsatz, bei welcher lokaler Druck auf das venöse Beingefäßsystem zu einer Steigerung der Fließgeschwindigkeit des Blutes führt. Dieser Druck kann durch Bandagieren des Beines mit Kompressionsbinden oder durch spezielle Strümpfe erzeugt werden Die Kompressionstherapie wird bei verschiedenen Krankheitsbildern eingesetzt, wie beispielsweise Chronisch venöse Insuffizienz, Postthrombotisches Syndrom, primäres und sekundäres Lymphödem, primäre und sekundäre Varikosis, Thrombophlebitis etc. Viele dieser Indikationen bergen neben dem Bedarf, Flüssigkeiten (beispielsweise odematöse Interstitialflüssigkeit) aufzunehmen, auch ein hohes Infektionsrisiko. Hier kommt der Kombination aus Kupfer bzw. Kupferionen und Superabsorbierenden Polymeren ein erhebliches Potential zu.

## Patentansprüche

1. Wundauflage zur Behandlung von chronischen oder Verbrennungswunden, aufweisend superabsorbierende Fasern aus Polyacrylaten, die mit Kupfer oder Kupferionen versehen oder imprägniert sind.

2. Wundauflage zur Behandlung von chronischen oder Verbrennungswunden gemäß Anspruch 1, **dadurch gekennzeichnet, dass** es sich bei den Kupferionen um Kupfer(I)- und/oder Kupfer(II)-Ionen handelt.

3. Wundauflage zur Behandlung von chronischen oder Verbrennungswunden gemäß Anspruch 1 oder 2, **dadurch gekennzeichnet, dass** es sich bei den Kupferionen um gebundene Kupferionen handelt, beispielsweise Kupfersalz, wobei die gebundenen Kupferionen als Verbindung mit einem anderen Stoff vorliegen und somit nach außen keine Ladung tragen.

4. Wundauflage zur Behandlung von chronischen oder Verbrennungswunden gemäß Anspruch 3, **dadurch gekennzeichnet, dass** es sich bei den gebundenen Kupferionen um Kupfersulfat, Kupferoxychlorid, Kupferchlorid und/oder Kupferhydroxid handelt.

5. Wundauflage zur Behandlung von chronischen oder Verbrennungswunden gemäß Anspruch 3, **dadurch gekennzeichnet, dass** es sich bei den gebundenen Kupferionen um Kupferoxid handelt.

6. Wundauflage zur Behandlung von chronischen oder Verbrennungswunden gemäß Anspruch 5, **dadurch gekennzeichnet, dass** das Kupferoxid in Teilchen mit einer Größe zwischen 0,5µm und 20µm vorliegt, besonders bevorzugt in einer Größe zwischen 1µm und 5µm.

7. Wundauflage zur Behandlung von chronischen oder Verbrennungswunden gemäß einem der vorherigen Ansprüche, **dadurch gekennzeichnet, dass** der Kupfer bzw. die Kupferionen in Pulverform, Granulatform, in Drahtform, als Kolloid, als Faserimprägnierung, und/oder als Späne vorliegen.

8. Wundauflage zur Behandlung von chronischen oder Verbrennungswunden gemäß einem der vorhergehenden Ansprüche, **dadurch gekennzeichnet, dass** er einen Materialabschnitt aufweisend superabsorbierende Polymere und eine mindestens abschnittsweise um diesen Materialabschnitt angeordnete Hülle aufweist.

9. Wundauflage zur Behandlung von chronischen oder Verbrennungswunden gemäß einem der vorhergehenden Ansprüche, **dadurch gekennzeichnet, dass** der Hygiene- oder Pflegeartikel weiterhin aufweist
a) einen Abschnitt aufweisend Weichschaumstoff,
b) ein Wunddistanzgitter, und/oder
c) eine Wunddrainageeinrichtung zum Einsatz bei Unterdruck.

10. Wundauflage zur Behandlung von chronischen oder Verbrennungswunden gemäß einem der vorherigen Ansprüche zur Verwendung als Kranken- und/oder Liegeunterlage.

11. Wundauflage zur Behandlung von chronischen oder Verbrennungswunden gemäß einem der vorherigen Ansprüche zur Verwendung als Kompressionsbandage.

12. Zusammensetzung aufweisend
a) superabsorbierende Polymerfasern auf Basis von Acrylaten, und
b) einen Anteil an Kupfer bzw. Kupferionen
zur Verwendung in der Behandlung von Wunden, insbesondere chronischen Wunden.

## Claims

1. A wound dressing for the treatment of chronic wounds or burns, comprising superabsorbent fibers of polyacrylates that are provided or impregnated with copper or copper ions.

2. The wound dressing for the treatment of chronic wounds or burns according to claim 1, **characterized in that** the copper ions are copper(I) and/or copper(II) ions.

3. The wound dressing for the treatment of chronic wounds or burns according to claim 1 or 2, **characterized in that** the copper ions are bonded copper ions, for example, copper salt, whereby the bonded copper ions are present in the form of a compound with another substance and thus have no charge towards the outside.

4. The wound dressing for the treatment of chronic wounds or burns according to claim 3, **characterized in that** the bonded copper ions are copper sulfate, copper oxychloride, copper chloride and/or copper hydroxide.

5. The wound dressing for the treatment of chronic wounds or burns according to claim 3, **characterized in that** the bonded copper ions are copper oxide.

6. The wound dressing for the treatment of chronic wounds or burns according to claim 5, **characterized in that** the copper oxide is present in particles having a size between 0.5 µm and 20 µm, especially preferably a size between 1 µm and 5 µm.

7. The wound dressing for the treatment of chronic wounds or burns according to one of the preceding claims, **characterized in that** the copper or the copper ions are present in powder form, in granule form, in wire form, as a colloid, as a fiber impregnation and/or as chips.

8. The wound dressing for the treatment of chronic wounds or burns according to one of the preceding claims, **characterized in that** it has a material section comprising superabsorbent polymers and a sheathing arranged around this material section, at least in certain areas.

9. The wound dressing for the treatment of chronic wounds or burns according to one of the preceding claims, **characterized in that** the hygiene or care article also has
a) a section comprising soft foam,
b) a wound spacer lattice, and/or
c) a wound drainage means for use at negative pressure.

10. The wound dressing for the treatment of chronic wounds or burns according to one of the preceding claims, for use as an undersheet and/or draw sheet.

11. The wound dressing for the treatment of chronic wounds or burns according to one of the preceding claims, for use as a compression bandage.

12. A composition comprising
a) superabsorbent polymer fibers on the basis of acrylates, and
b) an amount of copper or copper ions
for use in the treatment of wounds, especially chronic wounds.

## Revendications

1. Pansement pour le traitement de blessures chroniques ou de brûlures, comportant des fibres de polyacrylates superabsorbantes pourvues ou imprégnées de cuivre ou d'ions de cuivre.

2. Pansement pour le traitement de blessures chroniques ou de brûlures selon la revendication 1, **caractérisé en ce que** les ions de cuivre sont des ions de cuivre (I) et/ou des ions de cuivre (II).

3. Pansement pour le traitement de blessures chroniques ou de brûlures selon la revendication 1 ou 2, **caractérisé en ce que** les ions de cuivre sont des ions de cuivre liés, par exemple, du sel de cuivre, les ions de cuivre liés se présentant sous la forme d'une combinaison avec une autre substance et ne véhiculant ainsi pas de charge vers l'extérieur.

4. Pansement pour le traitement de blessures chroniques ou de brûlures selon la revendication 3, **caractérisé en ce que** les ions de cuivre liés sont du sulfate de cuivre, de l'oxychlorure de cuivre, du chlorure de cuivre et/ou de l'hydroxyde de cuivre.

5. Pansement pour le traitement de blessures chroniques ou de brûlures selon la revendication 3, **caractérisé en ce que** les ions de cuivre liés sont de l'oxyde de cuivre.

6. Pansement pour le traitement de blessures chroniques ou de brûlures selon la revendication 5, **caractérisé en ce que** l'oxyde de cuivre se présente sous la forme de particules ayant une taille comprise entre 0,5 µm et 20 µm, et de manière particulièrement préférentielle une taille comprise entre 1 µm et 5 µm.

7. Pansement pour le traitement de blessures chroniques ou de brûlures selon l'une des revendications précédentes, **caractérisé en ce que** le cuivre se présente sous la forme d'une poudre, sous la forme de granulés, sous la forme d'un fil, en tant que colloïde, en tant qu'imprégnation de fibre et/ou en tant que copeaux.

8. Pansement pour le traitement de blessures chroniques ou de brûlures selon l'une des revendications précédentes, **caractérisé en ce qu'**il présente une partie de matière comportant des polymères superabsorbants et une enveloppe agencée au moins en partie autour de cette partie de matière.

9. Pansement pour le traitement de blessures chroniques ou de brûlures selon l'une des revendications précédentes, **caractérisé en ce que** l'article hygiénique ou de soins comporte en outre :
a) une partie comportant une mousse souple,
b) une grille d'espacement de la plaie et/ou
c) un dispositif de drainage de plaie destiné à être mis en oeuvre sous dépression.

10. Pansement pour le traitement de blessures chroniques ou de brûlures selon l'une des revendications précédentes, destiné à être utilisé en tant qu'alèse pour malades et/ou pièce de literie.

11. Pansement pour le traitement de blessures chroniques ou de brûlures selon l'une des revendications précédentes destiné à être utilisé en tant que bandage de compression.

12. Composition comportant :
a) des fibres polymères superabsorbantes à base d'acrylates et
b) une fraction de cuivre ou d'ions de cuivre,
destinée à être utilisée pour le traitement de plaies, et plus particulièrement de plaies chroniques.
